# EUROPEAN PATENT APPLICATION

(11) **EP 4 794 302 A1**
(43) Date of publication of application: **19.08.2026**
(21) Application number: 25157850.6
(22) Date of filing: 14.02.2025
(51) Int. Cl.: H04L 67/12

(54) **CONFIGURING A DATA COLLECTION PLATFORM ADAPTED TO COLLECT ENVIRONMENT DATA**

(71) Applicant: OTT Hydromet Corp., Sterling, VA 20164 (US)
(72) Inventor: Rozitis, Andrejs, Louisville, CO 80027 (US); Koch, Greg, Fort Collins, CO 80525 (US); Nelson, Michael R, Leesburg, VA 20175 (US); Denton, Gretchen, Fort Lauderdale, FL 33311-7410 (US); Yanaga, Joanna Rita, Fort Collins, CO 80525 (US)
(74) Representative: Müller-Boré & Partner Patentanwälte PartG mbB

(57) **Abstract**

A method for configuring a data collection platform adapted to collect environment data obtained by one or more monitoring devices, the data collection platform comprising a processor and a communication interface for communicating with the monitoring devices according to a communication protocol, the method comprising: receiving an instruction to configure a new monitoring device to communicate with the data collection platform; automatically identifying an address assigned to each of the one or more monitoring devices, the one or more monitoring devices comprising the new monitoring device; sending, to the user computing device, the identified address or addresses assigned to the one or more monitoring devices; displaying, by the user computing device, a first graphical user interface, GUI, including: the identified addresses assigned to the monitoring devices, and information to prompt a user to change the address assigned to the new monitoring device; receiving user input to assign a new address to the new monitoring device; and assigning the new address to the new monitoring device.

## Description

The application relates to a data collection system, a data collection platform and a user computing device as well as methods and computer program products for configuring the data collection platform adapted to collect environment data.

### BACKGROUND

Data collection systems are widely used in many different fields and applications. Examples may include data collection systems employed for monitoring e.g. environmental parameters (such as meteorological, hydrological, road, and/or solar parameters), or other parameters or data. These applications oftentimes are set up at remote locations, where a data collection platform, sometimes also referred to as a data recorder or data logger, is connected to a plurality of measuring or monitoring devices, such as for example being or comprising one or more sensors for air temperature, air pressure, humidity, wind speed, water level, water quality, road surface conditions, solar radiation, and/or sun position, etc.

Measuring devices of many manufacturers for environmental and meteorological monitoring purposes oftentimes utilize a communication protocol referred to as SDI-12 (Serial Digital Interface at 1200 baud). Specifically, SDI-12 is an asynchronous serial communications protocol for measuring devices, in particular intelligent sensors that monitor environment data. These instruments are typically low-power (12 volts), are used at remote locations. The protocol follows a client-server configuration whereby a data logger (SDI-12 recorder) requests data from the intelligent sensors (SDI-12 sensors), each identified with a unique address. SDI-12 protocol may use the SDI-12 or RS-485 physical interfaces.

Large monitoring applications may oftentimes utilize a large number of diverse measuring devices, such as e.g. several tens or a few hundred measuring devices. This in turn oftentimes results in the initial setup and configuration of such monitoring applications may end up cumbersome and error prone. Notably, although it is quite easy to physically connect the measuring devices to one or more communication ports, in particular SDI-12, of a data collection platform, the configurations necessary to appropriately configure the large number of measuring devices with correct addresses, and to appropriately configure the data collection platform with appropriate parameter settings for each connected measuring device is quite laborious and error prone.

For example, with SDI-12, each monitoring device has (and requires) its own unique address number (with respect to the SDI-12 port it is connected to; different SDI-12 ports are separate from each other and therefore may see mutually duplicated addresses). At the same time, measuring devices, in particular intelligent sensors, are shipped by many manufacturers with a default address setting of 0. One approach that is used in the prior art is to either (1) use a custom configuration software to change the measuring device's SDI-12 address to a unique number before connecting it to the SDI-12 bus, or (2) have the user learn and issue custom SDI-12 commands to change the measuring device's address to a unique number. Both of these approaches are cumbersome and error prone.

There exists thus a need for a method that allows for configuring data collection systems with a large number of measuring devices connected to a data collection platform in a more simple, more easy and less error prone way. In particular, it is desirable to not require a user to possess technical knowledge of SDI-12 commands in order to effect a successful configuration. It is also desirable to speed up the configuration of large numbers of measuring devices.

### SUMMARY

According to an aspect, the problem relates to providing a data collection system, a data collection platform and a user computing device as well as methods and computer program products for configuring the data collection platform that allow for simpler and faster configuration, in particular for multiple monitoring devices, thereby particularly lowering the amount of technical knowledge and experience a user is required to possess in order to successfully configure the data collection system, and/or making configuration less prone for errors, also reducing need for subsequent troubleshooting.

The problem is solved by the features disclosed by the independent claims. Further exemplary embodiments are defined by the dependent claims.

According to a first aspect, there is provided a method for configuring a data collection platform adapted to collect environment data (such as meteorological, hydrological, road, and/or solar parameters) obtained by one or more monitoring devices, the data collection platform comprising a processor and a communication interface for communicating with the one or more monitoring devices according to a communication protocol, the method comprising: receiving, by the processor from a user computing device in communication with the data collection platform, an instruction to configure a new monitoring device to communicate with the data collection platform; automatically identifying, by the processor via the communication interface according to the communication protocol, an address assigned to each of the one or more monitoring devices, the one or more monitoring devices comprising the new monitoring device; sending, by the processor to the user computing device, the identified address or addresses assigned to the one or more monitoring devices; displaying, by the user computing device, a first graphical user interface, GUI, including: the identified address or addresses assigned to the one or more monitoring devices, and information to prompt a user to change the address assigned to the new monitoring device; receiving, by the user computing device via the first GUI, a user input to assign a new address to the new monitoring device; sending, by the user computing device to the processor, the user input indicating the new address; and assigning, by the processor via the communication interface according to the communication protocol, the new address to the new monitoring device.

Optionally, the new monitoring device initially is provided as having a default address being assigned to the new monitoring device, and the user is prompted to change the address assigned to the new monitoring device to an address that is different from the default address and that is different from any other monitoring device, wherein the default address preferably is 0.

The method may optionally further comprise: displaying, by the user computing device, a second GUI allowing the user to set configuration for the new monitoring device, the configuration including settings for one or more operations to be performed by the processor with respect to the new monitoring device; receiving, by the user computing device via the second GUI, the configuration set by the user for the new monitoring device; and sending the received configuration from the user computing device to the data collection platform. The method may optionally further comprise also: prior to the displaying of the second GUI, obtaining, by the user computing device from a database, a template of configuration for the new monitoring device, wherein the template comprises default settings of the configuration for the new monitoring device, and wherein the second GUI shows the default settings included in the template. Further preferably, the default settings comprised in the template include settings for power control operations of the new monitoring device, specifying timings for turning on and/or off the new monitoring device.

In each of the above mentioned methods, preferably, the identifying of the address assigned to each of the one or more monitoring devices may comprises: enabling power to the communication interface; for each one of a plurality of possible addresses: sending, via the communication interface according to the communication protocol, a request to return identification information of a monitoring device connected to the communication interface under said one of the plurality of possible addresses; receiving, via the communication interface according to the communication protocol, a reply to the request; in case the reply includes the identification information of the monitoring device, determining that said one of the plurality of possible addresses is the address assigned to the monitoring device; and in case the reply does not include the identification information, determining that said one of the plurality of addresses is assigned to no monitoring device, and further optionally comprising: after all of the possible addresses have been processed, disabling power to the communication interface.

In each of the above mentioned methods, preferably, the assigning of the new address to the new monitoring device may comprise: enabling power to the communication interface; sending, via the communication interface according to the communication protocol, a command to change the address identified for the new monitoring device to the new address, and further optionally, after having send the command to change the address, disabling power to the communication interface.

The communication protocol may be a serial communications protocol for intelligent sensors that monitor environment data, and the communication protocol may preferably be: Serial Digital Interface at 1200 baud, SDI-12, Modbus, or Universal Measurement Bus, UMB.

In a second aspect, there is provided a method performed by a processor of a data collection platform for configuring the data collection platform, the data collection being adapted to collect environment data (such as meteorological, hydrological, road, and/or solar parameters) obtained by one or more monitoring devices, the data collection platform comprising the processor and a communication interface for communicating with the one or more monitoring devices according to a communication protocol, the method comprising: receiving, from a user computing device in communication with the data collection platform, an instruction to configure a new monitoring device to communicate with the data collection platform; identifying, via the communication interface according to the communication protocol, an address of each of the one or more monitoring devices, the one or more monitoring devices comprising the new monitoring device; sending, to the user computing device, the identified address or addresses assigned to the one or more monitoring devices, thereby causing the user computing device to display a first graphical user interface, GUI, including: the identified address or addresses assigned to the one or more monitoring devices, and information to prompt a user to change the address assigned to the new monitoring device; receiving, from the user computing device, a user input received by the user computing device via the first GUI, the user input indicating a new address to be assigned to the new monitoring device; and assigning, via the communication interface according to the communication protocol, the new address to the new monitoring device.

In a third aspect, there is provided a computer program product comprising computer-readable instructions that, when loaded and run on a processor comprised in a data collection platform, cause the processor to perform the method according to the second aspect.

In a fourth aspect, there is provided a method performed by a user computing device for configuring a data collection platform adapted to collect environment data (such as meteorological, hydrological, road, and/or solar parameters) obtained by one or more monitoring devices, the user computing device being configured to communicate with the data collection platform, the method comprising: sending, to the data collection platform, an instruction to configure a new monitoring device to communicate with the data collection platform; receiving, from the data collection platform, one or more identified addresses assigned to the one or more monitoring devices that comprise the new monitoring device; displaying a first graphical user interface, GUI, including: the one or more identified addresses assigned to the one or more monitoring devices, and information to prompt a user to change the address assigned to the new monitoring device; receiving, via the first GUI, a user input to assigning a new address to the new monitoring device; sending, to the data collection platform, the user input indicating the new address, thereby causing the data collection platform to assign the new address to the new monitoring device.

In a fifth aspect, there is provided computer program product comprising computer-readable instructions that, when loaded and run on a computer, cause the computer to perform the method according to the fourth aspect.

In a sixth aspect, there is provided a data collection system comprising: one or more monitoring devices configured to obtain environment data (such as meteorological, hydrological, road, and/or solar parameters); a data collection platform adapted to collect the environment data obtained by the one or more monitoring devices; and a user computing device configured to communicate with the data collection platform, wherein the data collection platform comprises: a communication interface for communicating with the one or more monitoring devices according to a communication protocol; and a processor configured to: receive, from the user computing device, an instruction to configure a new monitoring device to communicate with the data collection platform; identify, via the communication interface according to the communication protocol, an address assigned to each of the one or more monitoring devices, the one or more monitoring devices comprising the new monitoring device; send, to the user computing device, the identified address or addresses assigned to the one or more monitoring devices; receive, from the user computing device, a user input indicating a new address to be assigned to the new monitoring device; and assign, via the communication interface according to the communication protocol, the new address to the new monitoring device; and wherein the user computing device is configured to: display, in response to receiving the identified address or addresses from the processor of the data collection platform, a first graphical user interface, GUI, including: the identified address or addresses assigned to the one or more monitoring devices, and information to prompt a user to change the address assigned to the new monitoring device; receive, via the first GUI, the user input to assign the new address to the new monitoring device; and send, to the processor of the data collection platform, the user input indicating the new address.

In a seventh aspect, there is provided a data collection platform adapted to collect the environment data (such as meteorological, hydrological, road, and/or solar parameters) obtained by the one or more monitoring devices, the data collection platform comprising: a communication interface for communicating with the one or more monitoring devices according to a communication protocol; and a processor configured to: receive, from a user computing device in communication with the data collection platform, an instruction to configure a new monitoring device to communicate with the data collection platform; identify, via the communication interface according to the communication protocol, an address assigned to each of the one or more monitoring devices, the one or more monitoring devices comprising the new monitoring device; send, to the user computing device, the identified address or addresses assigned to the one or more monitoring devices; receive, from the user computing device, a user input indicating a new address to be assigned to the new monitoring device; and assign, via the communication interface according to the communication protocol, the new address to the new monitoring device.

In an eight aspect, there is provided a user computing device configured to communicate with a data collection platform adapted to collect environment data (such as meteorological, hydrological, road, and/or solar parameters) obtained by one or more monitoring devices, the user computing device comprising: one or more processors configured to: send, to the data collection platform, an instruction to configure a new monitoring device to communicate with the data collection platform; receive, from the data collection platform, one or more identified addresses assigned to the one or more monitoring devices; display a first graphical user interface, GUI, including: the one or more identified addresses assigned to the one or more monitoring devices, and information to prompt a user to change the address assigned to the new monitoring device; receive, via the first GUI, the user input to assign the new address to the new monitoring device; and send, to the processor of the data collection platform, the user input indicating the new address, thereby causing the data collection platform to assign the new address to the new monitoring device.

The subject matter described in the application can be implemented as a method or as a system, possibly in the form of one or more computer program products. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. In some examples, the system may be a general purpose computer system. In other examples, the system may be a special purpose computer system including an embedded system.

### BRIEF DESCRIPTION OF THE DRAWINGS

Details of one or more implementations are set forth in the exemplary drawings and description below. Other features will be apparent from the description, the drawings, and from the claims. It should be understood, however, that even though embodiments are separately described, single features of different embodiments may be combined to further embodiments.
**Fig. 1** shows a data collection system according to an embodiment of the present disclosure;
**Fig. 2** shows an example of a method for configuring a data collection platform with at least one monitoring device according to an embodiment of the present disclosure;
**Fig. 3** shows a graphical user interface for inputting an address range to be scanned according to an embodiment of the present disclosure;
**Fig. 4** shows details of an automatic identification procedure according to an embodiment of the present disclosure;
**Fig. 5a to 5c** respectively show a graphical user interface for changing an address of a measuring device.
**Fig. 6** shows details of a procedure to assign the new address to the new monitoring device according to an embodiment of the present disclosure;
**Fig. 7** shows a graphical user interface for configuring parameters and settings associated with a measuring device according to an embodiment of the present disclosure; and
**Fig. 8** shows an exemplary hardware configuration of a computer that may be used to implement at least a part of a system according to an embodiment of the present disclosure;

### DETAILED DESCRIPTION OF EMBODIMENTS

In the following text, a detailed description of examples will be given with reference to the drawings. It should be understood that various modifications to the examples may be made. In particular, one or more elements of one example may be combined and used in other examples to form new examples.

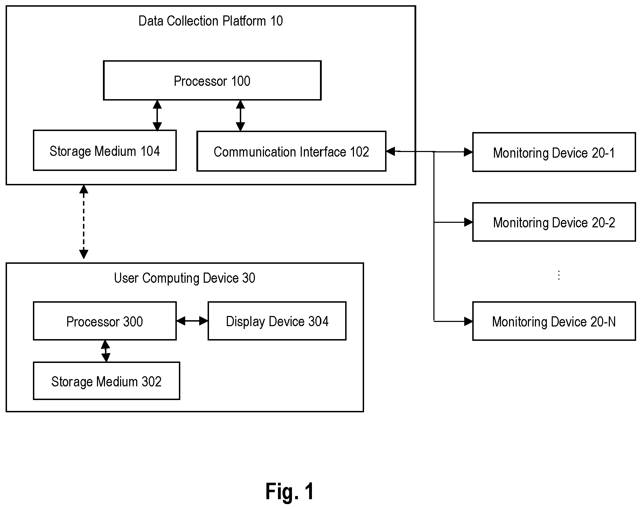

**Fig. 1** shows a data collection system also referred to as a data recorder or data logger according to an embodiment of the present disclosure adapted to collect environment data (particularly of meteorological meteorological, hydrological, road, and/or solar data). Specifically, the data collection system records data over time and/or about one or more locations, processes the collected data and/or facilitates data transmission e.g. via WiFi, bluetooth, low energy bluetooth (BLE), an external cellular modem or the like.

As shown in Fig. 1, the data collection system comprises a data collection platform 10 that comprises a processor 100, a storage medium 104 and/or at least one communication interface 102 implementing a communication protocol.

The storage medium 104 may store program code that, when executed by the processor 100, cause the processor 100 to perform the various steps described herein related to the data collection platform 10.

A plurality of monitoring devices 20-1, 20-2, ..., 20-N (collectively referred to as monitoring device(s) 20) are connected to the communication interface 102 of the data collection platform 10.

Specifically, the monitoring device(s) 20 may comprise or be one or more sensors for determining environmental data or parameters relating to air temperature, barometric pressure, precipitation quantity (particularly including cumulative precipitation, precipitation intensity and/or bucket content specifically in real time and/or non-real time), precipitation type (e.g. rain, snow, sleet, freezing rain and/or hail), precipitation drop size distribution, radar reflectivity, humidity (particularly relative air humidity and/or absolute air humidity), dewpoint, wind speed, wind direction, water level, water flow data, and/or water quality, Specifically, the one or more sensors may determine temperature, conductivity, depth, pressure, pH, dissolved oxygen (particularly luminescent dissolved oxygen, LDO), turbidity, Oxidation-reduction potential (ORP), Clorophyll a, Blue-green algae, Rhodamine, Ammonium, Nitrate and/or Chloride. Further particularly, the monitoring device(s) 20 may comprise or be one or more sensors for determining environmental data or parameters relating to a precipitation drop size distribution, a precipitation radar reflectivity, a solar radiation, lightning, a road condition (e.g. determine or categorize whether the road condition dry, moist, wet, ice, snow, slush and/or chemically wet), a road surface temperature, a water film height, a road friction (e.g. calculated), a runway condition code (RCC), a road ice percentage, a road/runway water/ice/snow layer thickness, a freezing temperature for different de-icing materials (e.g. NaCl, MgCl, CaCl), a snow depth, an aerosol backscatter, an aerosol layer height, a cloud cover, a cloud base height, a cloud penetration depth, a vertical visibility, a sky condition index, a visibility, an albedo, a solar irradiance, a sunshine duration, an infrared radiation, a sun position, an UV irradiance/radiation, an ozone presence or quantity, SO2, one or more UV spectra, an atmospheric turbulence, a photovoltaic panel dust/sand/pollen and/or photovoltaic panel temperature.

As an example, the monitoring device(s) may comprise any one or more of the following sensors (particularly of Ott Hydromet or Lufft): -a weather sensor of the WS series (WS200, WS300, WS400, WS500, WS600, WS700 and/or WS800), an anemometer, a pyranometer, a runway/road sensor (e.g. MARWIS, NIRS, PRS and/or ARS), -a snow depth sensor (SHM), a ceilometer (CHM), and/or a visibility sensor (e.g. VS2k or VS20k).

As a further example, the monitoring device(s) may comprise any one or more of the following sensors (particularly of Kipp & Zonen): an albedometer, an irradiance sensor (e.g. RaZON+), one or more pyranometers (e.g. SP Lite2, CM4, CMP3, SMP3, CMP6, SMP6, CMP10, SMP10, SMP12, CMP21, CMP22 and/or SMP22), one or more pyrgeometers (CGR4 and/or SGR4), a pyrheliometer (e.g. CHP1 and/or SHP1), a sun tracker (e.g. SOLYS and/or SOLYS2), a UV radiometer (CUV and/or SUV), a net radiometer (e.g. NR Lite2 and/or CNR4), a sunshine duration sensor (e.g. CSD3), and/or a photovoltaic soiling sensor (e.g. Dust IQ).

As a still further example, the monitoring device(s) may comprise any one or more of the following sensors (particularly of Sutron): a constant flow bubbler, a SDR stage discharge recorder, a tipping bucket rain gauge,a soil moisture sensor, a barometric sensor, an AcquaTemp, and/or a Shaft encoder.

As an example, the monitoring device(s) may comprise any one or more of the following sensors (particularly of Ott Hydromet or Lufft): a Hydrolab HL4 or HL7 Multiparameter Sonde, Sea-Bird Scientific HydroCAT-EP, Sea-Bird Scientific SUNA Optical Nitrate Sensor, Sea-Bird Scientific HydroCAT, OTT PLS 500 Pressure Level Sensor, OTT ecoLog 800 or 1000 Water Level Logger, OTT RLS Radar Level Sensor, OTT SVR 100 Surface Velocity Radar, OTT C2 or C31 Universal Current Meter,OTT Pluvio² S or L Weighing Rain Gauge, OTT Parsivel² Laser Weather Sensor, Lufft WS100 Radar Precipitation Sensor/Smart Disdrometer, weather sensor OTT TRH, Lufft Ventus Ultrasonic Wind Sensor.

Moreover, the monitoring device(s) 20 may comprise or be one or more imaging devices such as a video camera.

The storage medium 104 may store measurement data obtained from the monitoring device(s) 20, e.g. by polling through the communication interface 102 one or more of the measuring devices 20 particularly according to a respective measuring interval configured in the data collection platform 10.

In some embodiments, the communication protocol implemented by the communication interface 102 may a serial communications protocol for intelligent sensors that monitor environment data. The communication protocol may for example be any one of Serial Digital Interface at 1200 baud (SDI-12), Modbus and Universal Measurement Bus (UMB).

As further shown in Fig. 1, a user computing device 30 may be connected to the data collecting platform 10 via a wired and/or wireless data connection. The user computing device 30 comprises a processor 300 and a storage medium 302.

The storage medium 302 may store program code that, when executed by the processor 300, cause the processor 300 to perform the various steps described herein related to the user computing device 30.

The user computing device 30 may comprise an output device such as a display 304 for outputting (e.g. presenting) information to a user, and further comprises input means, such as a keyboard, one or more buttons, a computer mouse or the like (not shown) for receiving and/or processing user input.

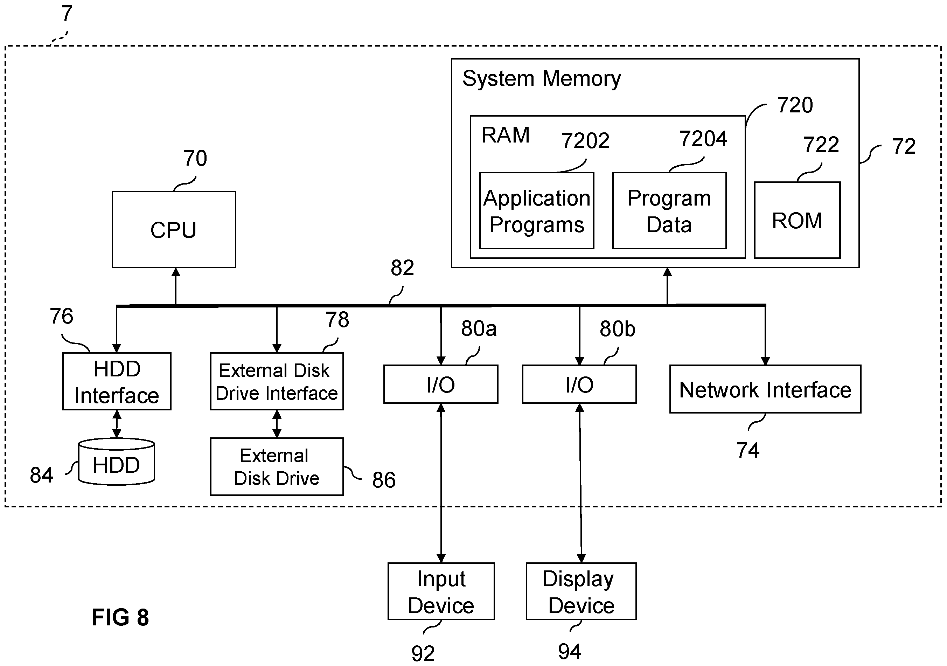

The data collection platform 10 may also comprise at least one output device such as a display (e.g. display device 94 shown in Fig. 8) for outputting/presenting information to the user, and/or one or more input means (such as input device 92 shown in Fig. 8), such as one or more buttons for receiving/processing user input.

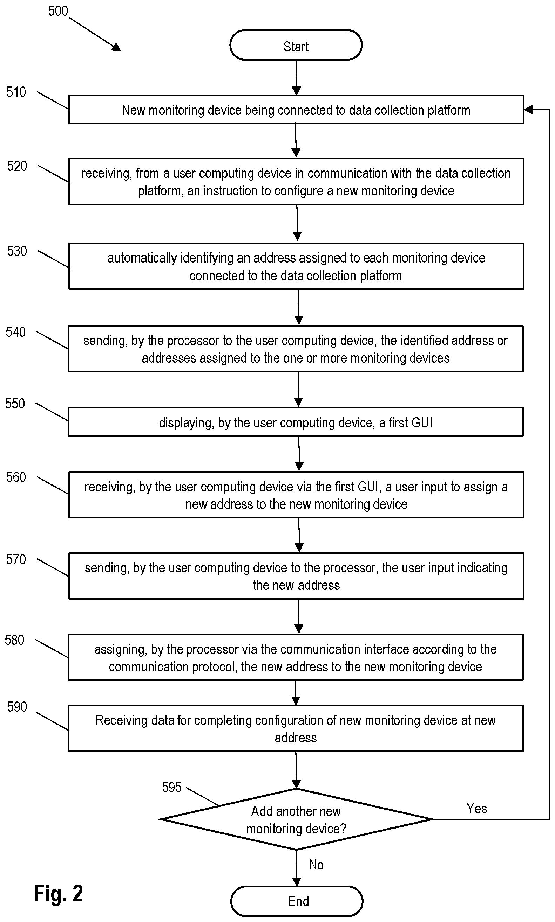

**Fig. 2** shows an example of a method 500 for configuring a data collection platform 10 adapted to collect environment data with at least one monitoring device 20. In the example of Fig. 2 it is assumed that initially, there is not connected any monitoring device 20 to the data collection platform 10. The new monitoring device 20 may initially be configured with a default address value e.g. of 0.

The method starts, in step 510, with a new monitoring device 20 (e.g. the monitoring device 20-1) being connected to the communication interface 102 of the data collection platform 10. Here, the new monitoring device 20 particularly may be a SDI-12 monitoring device, such as e.g. a sensor for measuring any one or more of temperature, humidity or relative humidity, water level, water flow, water quality etc.

In step 520, the processor 100 of the data collection platform 10 receives, from a user computing device 20 that is connected to the data collection platform 10, one or more instructions to configure the new monitoring device 20 to communicate with the data collection platform 10.

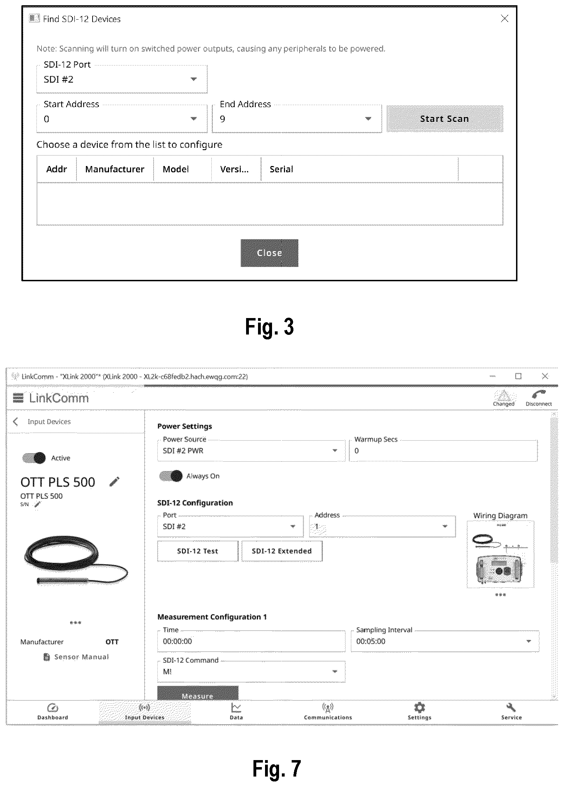

This may preferably be implemented by the user computing device 20 displaying to the user a graphical user interface (GUI), such as e.g. shown in **Fig. 3****,** where the user is presented with an option to select a communication interface 102 respectively a communication port that is to be scanned for detecting new measuring devices 20 connected to the communication interface 102.

In the example GUI of Fig. 3, the user has selected (e.g. from a pull-down menu) the "SDI-12 Port" indicated as "SDI-12 #2", i.e., the second SDI-12 port as a target for scanning.

The GUI moreover allows the user to specify a "Start Address" and an "End Address" to specify the range of addresses that should be (particularly iteratively or concurrently) scanned.

Once the user has appropriately configured these settings, the user may click on the button "Start Scan", to thereby effect the user computing device 20 to send a corresponding command to the proc 100 of the data collection platform 10.

Turning back to **Fig. 2****,** upon receiving this command, in step 530, the data collection platform 10 performs an automatic identification of which one or more monitoring devices 20 are connected to the selected communication interface 102, to thereby identify the address of each respective monitoring device 20 that is connected to the selected communication interface 102.

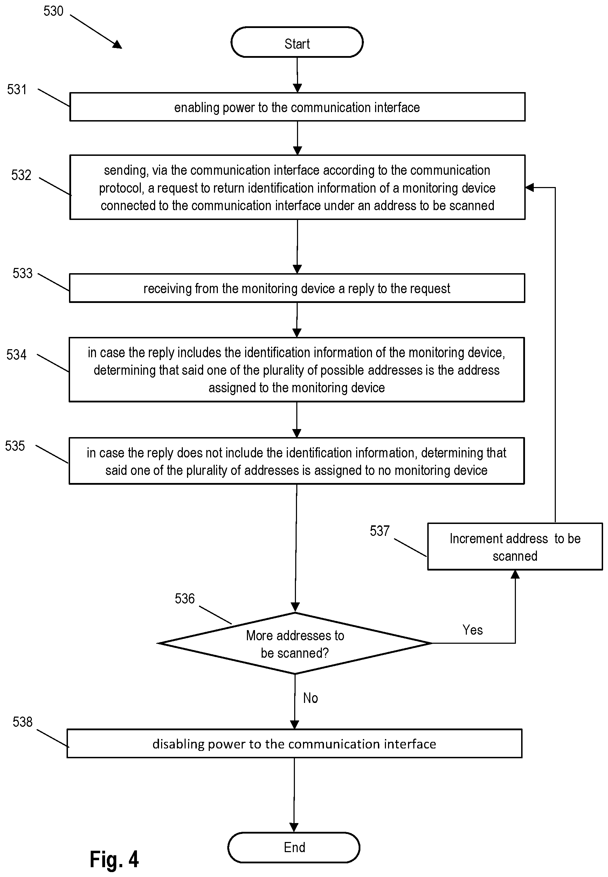

An example of an automatic identification procedure 530 will be explained with regard to **Fig. 4****.**

As shown in Fig. 4, the automatic identification procedure 530 starts with enabling power to the communication interface 102 in step 531 in order to power up the SDI-12 bus and moreover to supply the monitoring devices 20 with power.

In step 532, there is sent via the communication interface 102, a request to return identification information of a monitoring device 20 that is connected to the communication interface 102. The request is formed or configured according to the communication protocol and is sent to at least one of the plurality of addresses that are to be scanned.

As an example, the request may be a SDI-12 conform request that is addressed to address 0 as the start address that has been configured in the GUI shown in Fig. 3.

For example, the corresponding SDI-12 command may be:

| | | |
|---|---|---|
| Command: | T 0I! | Use Transparent mode (T) and request that monitoring device with address zero (0) sends information (I) |

A reply is then received in step 533. The reply may then be e.g.:

| | | | | |
|---|---|---|---|---|
| 0 | OTTHYDRO | PLS500 | 010 | PS5-0000 |

Address Manufacturer Model / Firmware / Serial Number

In case that there is not connected any monitoring device 20 with the corresponding address, then the request particularly will time out. Also such a time out may here be considered as a "reply".

In step 534, in case the reply includes the identification information of the monitoring device, it is determined that the address that has been scanned for is the address that is assigned to the monitoring device 20 that has sent the reply.

Otherwise, in step 535, in case the reply does not include the identification information, e.g. because the reply is a time out, it is determined that the address that has been scanned for is not assigned to any monitoring device 20.

In step 536, it is determined whether there are more addresses that need to be scanned, and if so, it is proceeded to step 537, where the address to be scanned is incremented and then returned to step 523.

If in step 536 it is determined that all addresses have been scanned, then the power to the communication interface 102 particularly is disabled in step 538 and the automatic identification procedure 530 ends.

Powering down the communication interface 102, and thus the SDI-12 bus, at this time is possible, as no further operations are required to be performed by the measuring devices 20 at this time, so that the SDI-12 bus and the measuring devices 20 may be powered down to particularly save electrical power and, thus, do not unnecessarily waste battery life.

Referring back to **Fig. 2****,** the information about the one or more addresses that have been identified in step 530 as being assigned to the one or more monitoring devices 20 connected to the communication interface 102 is then output, particularly sent to the user computing device 30, preferably together with respective information identifying the monitoring device 20.

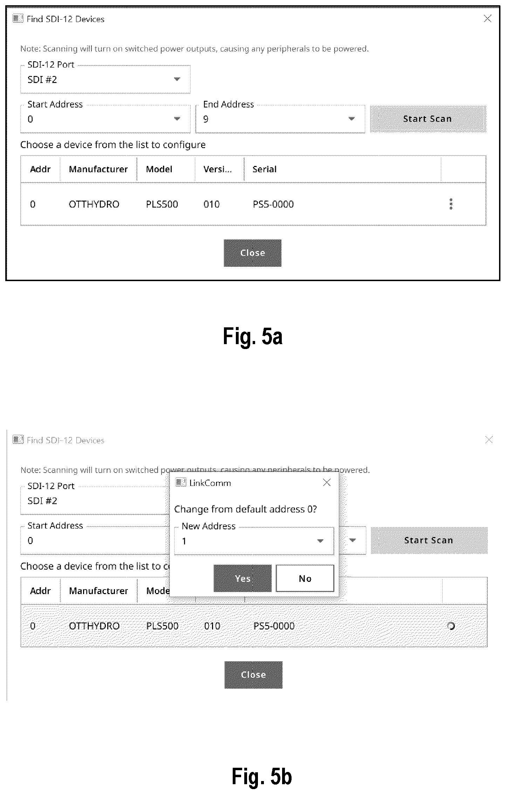

The user computing device 20 then outputs (e.g. displays), in step 550, to the user e.g. via a GUI including the identified address or addresses assigned to the one or more monitoring devices 20. This is shown e.g. in **Fig. 5a****,** where the identified new monitoring device 20 is shown to have been identified at address 0.

The GUI may also display information to prompt a user to change the address assigned to the new monitoring device. This is shown e.g. in **Fig. 5b****,** where the user is asked whether the address should be changed from the old, identified address (in this case: 0), and if so, to select a new address that the new monitoring device 20 should be configured with.

In this example, the user has selected the new address to be the address 1. When the user confirms the selection e.g. by clicking the "Yes" button, the corresponding selection is confirmed, and the user computing device 30 receives (particularly via this GUI) the user input to assign this new address to the new monitoring device 20 (step 560 in Fig. 2).

Continuing with **Fig. 2****,** the user computing device 30 then sends this user input indicating the new address to the data collection platform 10 in step 570.

In step 580, the data collection platform 10 then performs a procedure to assign the new address to the new monitoring device 20.

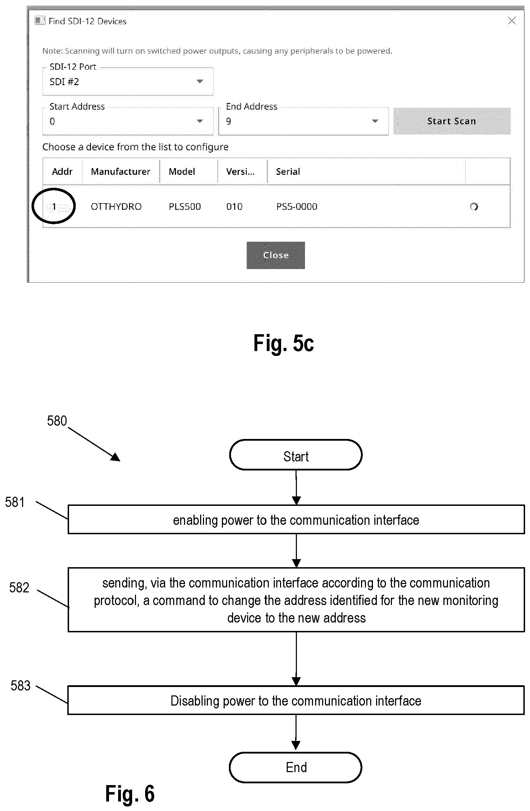

An example of a procedure 580 to assign the new address to the new monitoring device 20 will be explained with regard to **Fig. 6****.**

As shown in Fig. 6, the procedure 580 to assign the new address to the new monitoring device 20 starts in step 581 with enabling power to the communication interface 102 to thereby again power up the SDI-12 bus and the monitoring devices 20.

In Step 582, there Is sent, via the communication interface 102 and according the communication protocol, a command to change the address identified for the new monitoring device to the new address selected by the user. In the example of Fig. 5c, where the address is to be changed from address 0 to address 1, a corresponding SDI-12 command may be:

| | |
|---|---|
| Command: | 0A1! |

After the command has been issued and effected, in step 583, power to the communication interface 102 particularly is again disabled so that the SDI-12 bus and the measuring devices 20 may be powered down to save electrical power and thus do not unnecessarily waste battery life.

Referring back to Fig. 2, in step 590, the data collection platform 10 receives data and/or instructions for completing the configuration of the new monitoring device 20 at the new address.

To this purpose, the user particularly is presented, via the display of the user computing device 30, with a GUI, such as e.g. shown in **Fig. 7****,** that allows to configure the various aspects and/or settings relating to the new measuring device 20., As an example, the user is allowed to determine the sampling interval that is to be applied with regard to this measuring device 20.

Accordingly, the user particularly can select the SDI-12 command that is to be sent by the data collection platform 10 to the measuring device 20 in order to instruct the latter to perform a measurement and to send back the corresponding measurement value, and/or settings regarding whether the measuring device 20 is powered via the SDI-12 bus or not.

Here, it is noted that because the address assigned to the newly added measuring device 20 has already been changed from the initial value (the default value of 0) to the intended, user selected value of 1, this GUI is displayed with respect to the correct measuring device address that is intended to be used for regular data monitoring and collection operation purposes. In this way, the risk of unintentionally and erroneously configuring the "wrong" measuring device 20 due to not having set the measuring device address correctly can be reduced, thereby reducing risk of having to perform troubleshooting due to misconfigurations. Also, address collision of the measuring devices 20 can be advantageously avoided and/or the process of configuration is substantially simplified.

When displaying the configuration GUI, the one or more parameters and/or settings that may be configured for the respective monitoring device 20 may be at least partly preselected with respective default values. These respective default values may be provided with the GUI and may be irrespective of the type, manufacturer, etc. of the measuring device 20 to be configured.

Preferably, however, the data collection platform 10 and/or the user computing device 20 may be provided with one or more templates, where each template is associated with a measuring device model, and optionally manufacturer and has recorded or preset one or more default values for the parameters (e.g. to be presented via the GUI of Fig. 7) that may be the most appropriate and/or most often used values for this model and manufacturer type of measuring device.

The identification information of the monitoring device 20 returned in step 532 may be utilized to identify the appropriate template. If such a template is available, the configuration GUI as shown in Fig. 7 may be preset to the default values provided by the template. In this way, many settings and configurations will be adjusted in an automatic fashion so that a user will only rarely have to make manual changes. In this way, configuration can be simplified even more, and risks of misconfigurations can be reduced even more.

The template may contain different settings that define a typical configuration for a specific measuring device 20 respectively sensor. For example when a specific model is identified/selected, the available parameters are added to the GUI without the user having to discover and configure those manually.

Templates may contain one or more different parameters that can be made or set, e.g. temperature and level for OTT PLS. Templates may be provided/supported also e.g. for one or more cameras as examples of the monitoring device(s) 20.

Templates may be stored as JSON formatted entries in a database. Templates preferably contain measuring device-type specific optimized power setting configurations with optimized turn-on/turn-off delays.

Also, because the configuration GUI of Fig. 7 in this way can be provided by default (either in general or by means of model/manufacturer specific measuring device templates) with the appropriate SDI-12 commands, the user is relieved of the burden of having to know these commands and having to manually type and input these commands as would be required in a simple terminal-like application. Also in this way, configuration can be simplified even more, and/or risks of misconfigurations can be reduced even more.

The configuration for the entire data collection platform 10 can also be stored and/or loaded again as a configuration file.

The configuration file can be extracted from the data collection platform 10 over a variety of configuration interfaces such as USB, Ethernet, WiFi, Bluetooth, and/or exported from the configuration software (e.g. LinkComm of OTT HydroMet).

The configuration file can be organized in a common format such as JSON, XML. The configuration file can be shared by one or more electronic means such as email, FTP, a network-attached storage, etc.

The configuration file can be modified directly by a text editor and/or imported to configuration software (e.g. LinkComm) for offline editing when not directly connected to the device or directly connected.

The configuration file may contain settings for the data collection platform 10 and/or the one or more monitoring devices 20 to perform one or more of the following tasks/processes:
- Set electrical interfaces to interact with connected devices 20 (such as sensors, communication devices, cameras);
- Set communication parameters for specific protocols such as SDI-12, Modbus, UMB, HTTP, FTP, MQTT and/or SMTP;
- Sample input devices (particularly measuring device(s) 20) and at specific intervals to gather observation/measurement/sensor data;
- Dynamically control power to connected devices (particularly measuring device(s) 20) for the purpose of reducing overall power consumption by turning off when not in use, with appropriate turn-on and turn-off delays;
- Process data by adding significant digits, decimal points, measurement units (m, cm, psi, etc.), offset, scale, look-up tables (in the case of platinum RTD resistance-to-temperature), conversion (number of digital pulses to wind speed);
- Perform one or more calculations on data after ingesting observations/measurements from input devices (particularly from measuring device(s) 20);
- Accumulate data from multiple consecutive samples (e.g. rainfall across an hour, day, week) and reset accumulation according to a schedule;
- Setup triggers on data sources, and take action such as sending a notification or changing its own operation, e.g. increase sample frequency;
- Store data internally (e.g. in the storage medium 104), and/or on a connected storage device, and/or transmit to remote servers e.g. over SMTP email, FTP, MQTT, Satellite;
- Accept remote connections specifically only during specific, pre-defined days/times to increase cybersecurity and/or reduce the amount of time communication ports are open to outside connection;
- Set data collection platform 10 time according to internal real-time clock, or network time server, apply time-zone and/or apply daylight savings offset.

In step 595, it is determined whether another new monitoring device 20 should be added. This determination may comprise displaying to the user, at the user computing device 30, a GUI prompt asking whether a new monitoring device 20 should be added. If yes, the method returns to step 510. Otherwise, if no new monitoring device 20 is to be added, the method ends.

**Fig. 8** shows an exemplary hardware configuration of a computer that may be used to implement at least a part of the system as described above. For example, at least a part of the data collection platform 10 and/or the user computing device 30 shown in Fig. 1 may be implemented with the computer 7 shown in Fig. 8.

he computer 7 shown in Fig. 8 includes a central processing unit (CPU) 70, a system memory 72, a network interface 74, a hard disk drive (HDD) interface 76, an external disk drive interface 78 and input/output (I/O) interfaces 80. These components of the computer are coupled to each other via a system bus 82. The CPU 70 may perform arithmetic, logic and/or control operations by accessing the system memory 72. The system memory 72 may store information and/or instructions for use in combination with the CPU 70. The system memory 72 may include volatile and non-volatile memory, such as a random access memory (RAM) 720 and a read only memory (ROM) 722. A basic input/output system (BIOS) containing the routines that helps to transfer information between elements within the computer 7, such as during start-up, may be stored in the ROM 722. The system bus 82 may be any of several types of bus structures including a memory bus or memory controller, a peripheral bus, and a local bus using any of a variety of bus architectures.

The computer may include a network interface 74 for communicating with other computers and/or devices via a network.

Further, the computer may include a hard disk drive (HDD) 84 for reading from and writing to a hard disk (not shown), and an external disk drive 86 for reading from or writing to a removable disk (not shown). The removable disk may be a magnetic disk for a magnetic disk drive or an optical disk such as a CD ROM for an optical disk drive. The HDD 84 and the external disk drive 86 are connected to the system bus 82 by a HDD interface 76 and an external disk drive interface 78, respectively. The drives and their associated computer-readable media provide non-volatile storage of computer-readable instructions, data structures, program modules and other data for the general purpose computer. The data structures may include relevant data for the implementation of the exemplary method and its variations as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 722 or RAM 720, including an operating system (not shown), one or more application programs 7202, other program modules (not shown), and program data 7204. The application programs may include at least a part of the functionality as described above.

The computer 7 may be connected to an input device 92 such as mouse and/or keyboard and a display device 94 such as liquid crystal display, via corresponding I/O interfaces 80a and 80b as well as the system bus 82. In case the computer 7 is implemented as a tablet computer, for example, a touch panel that displays information and that receives input may be connected to the computer 7 via a corresponding I/O interface and the system bus 82. Further, in some examples, although not shown in Fig. 8, the computer 7 may further be connected to a printer and/or an imaging device such as a camera, via corresponding I/O interfaces and the system bus 82.

In addition or as an alternative to an implementation using a computer 7 as shown in Fig. 8, a part or all of the functionality of the exemplary embodiments described herein may be implemented as one or more hardware circuits. Examples of such hardware circuits may include but are not limited to: Large Scale Integration (LSI), Reduced Instruction Set Circuits (RISC), Application Specific Integrated Circuit (ASIC) and Field Programmable Gate Array (FPGA).

## Claims

1. A method (500) for configuring a data collection platform (10) adapted to collect environment data obtained by one or more monitoring devices (20), the data collection platform (10) comprising a processor (100) and a communication interface (102) for communicating with the one or more monitoring devices (20) according to a communication protocol, the method comprising:
receiving (520), by the processor (100) from a user computing device (30) in communication with the data collection platform (10), an instruction to configure a new monitoring device (20) to communicate with the data collection platform (10);
automatically identifying (530), by the processor (100) via the communication interface (102) according to the communication protocol, an address assigned to each of the one or more monitoring devices (20), the one or more monitoring devices (20) comprising the new monitoring device (20);
sending (540), by the processor (100) to the user computing device (30), the identified address or addresses assigned to the one or more monitoring devices (20);
displaying (550), by the user computing device (30), a first graphical user interface, GUI, including:
the identified address or addresses assigned to the one or more monitoring devices (20), and
information to prompt a user to change the address assigned to the new monitoring device (20);
receiving (560), by the user computing device (30) via the first GUI, a user input to assign a new address to the new monitoring device (20);
sending (570), by the user computing device (30) to the processor (100), the user input indicating the new address; and
assigning (580), by the processor (100) via the communication interface (102) according to the communication protocol, the new address to the new monitoring device (20).

2. The method (500) according to claim 1, further comprising:
displaying, by the user computing device (30), a second GUI allowing the user to set configuration for the new monitoring device (20), the configuration including settings for one or more operations to be performed by the processor (100) with respect to the new monitoring device (20);
receiving (590), by the user computing device (30) via the second GUI, the configuration set by the user for the new monitoring device (20); and
sending the received configuration from the user computing device (30) to the data collection platform (10).

3. The method (500) according to claim 2, further comprising:
prior to the displaying of the second GUI, obtaining, by the user computing device (30) from a database, a template of configuration for the new monitoring device (20),
wherein the template comprises default settings of the configuration for the new monitoring device (20), and
wherein the second GUI shows the default settings included in the template.

4. The method (500) according to claim 3, wherein the default settings comprised in the template include settings for power control operations of the new monitoring device (20), specifying timings for turning on and/or off the new monitoring device (20).

5. The method (500) according to any one of claims 2 to 4, wherein the received configuration is sent to the data collection platform (10) in a specified format, and
wherein the specified format may be JSON or XML.

6. The method (500) according to any one of the preceding claims, wherein the automatic identifying of the address assigned to each of the one or more monitoring devices (20) comprises:
enabling (531) power to the communication interface (102);
for each one of a plurality of possible addresses:
sending (532), via the communication interface (102) according to the communication protocol, a request to return identification information of a monitoring device (20) connected to the communication interface (102) under said one of the plurality of possible addresses;
receiving (533), via the communication interface (102) according to the communication protocol, a reply to the request;
in case the reply includes the identification information of the monitoring device (20), determining (534) that said one of the plurality of possible addresses is the address assigned to the monitoring device (20); and
in case the reply does not include the identification information, determining (535) that said one of the plurality of addresses is assigned to no monitoring device (20).

7. The method (500) according to any one of the preceding claims, wherein the assigning of the new address to the new monitoring device (20) comprises:
enabling (581) power to the communication interface (102); and
sending (582), via the communication interface (102) according to the communication protocol, a command to change the address identified for the new monitoring device (20) to the new address.

8. The method (500) according to any one of the preceding claims, wherein the communication protocol is a serial communications protocol for intelligent sensors that monitor environment data, and
wherein the communication protocol may be:
Serial Digital Interface at 1200 baud, SDI-12,
Modbus, or
Universal Measurement Bus, UMB.

9. A method (500) performed by a processor (100) of a data collection platform (10) for configuring the data collection platform (10), the data collection being adapted to collect environment data obtained by one or more monitoring devices (20), the data collection platform (10) comprising the processor (100) and a communication interface (102) for communicating with the one or more monitoring devices (20) according to a communication protocol, the method comprising:
receiving, from a user computing device (30) in communication with the data collection platform (10), an instruction to configure a new monitoring device (20) to communicate with the data collection platform (10);
automatically identifying, via the communication interface (102) according to the communication protocol, an address of each of the one or more monitoring devices (20), the one or more monitoring devices (20) comprising the new monitoring device;
sending, to the user computing device (30), the identified address or addresses assigned to the one or more monitoring devices (20), thereby causing the user computing device (30) to display a first graphical user interface, GUI, including:
the identified address or addresses assigned to the one or more monitoring devices (20), and
information to prompt a user to change the address assigned to the new monitoring device (20);
receiving, from the user computing device (30), a user input received by the user computing device (30) via the first GUI, the user input indicating a new address to be assigned to the new monitoring device (20); and
assigning, via the communication interface (102) according to the communication protocol, the new address to the new monitoring device (20).

10. A computer program product comprising computer-readable instructions that, when loaded and run on a processor (100) comprised in a data collection platform (10), cause the processor (100) to perform the method (500) according to claim 9.

11. A method performed by a user computing device (30) for configuring a data collection platform (10) adapted to collect environment data obtained by one or more monitoring devices (20), the user computing device (30) being configured to communicate with the data collection platform (10), the method comprising:
sending, to the data collection platform (10), an instruction to configure a new monitoring device (20) to communicate with the data collection platform (10), thereby causing the data collection platform (10) to automatically identify, via a communication interface (102) according to a communication protocol, an address of each of the one or more monitoring devices (20), the one or more monitoring devices (20) comprising the new monitoring device (20);
receiving, from the data collection platform (10), the identified address or addresses assigned to the one or more monitoring devices (20);
displaying a first graphical user interface, GUI, including:
the identified address or addresses assigned to the one or more monitoring devices (20), and
information to prompt a user to change the address assigned to the new monitoring device (20);
receiving, via the first GUI, a user input to assigning a new address to the new monitoring device (20);
sending, to the data collection platform (10), the user input indicating the new address, thereby causing the data collection platform (10) to assign the new address to the new monitoring device (20).

12. A computer program product comprising computer-readable instructions that, when loaded and run on a computer, cause the computer to perform the method according to claim 11.

13. Data collection system comprising:
one or more monitoring devices (20) configured to obtain environment data;
a data collection platform (10) adapted to collect the environment data obtained by the one or more monitoring devices (20); and
a user computing device (30) configured to communicate with the data collection platform (10),
wherein the data collection platform (10) comprises:
a communication interface (102) for communicating with the one or more monitoring devices (20) according to a communication protocol; and
a processor configured to:
receive, from the user computing device (30), an instruction to configure a new monitoring device (20) to communicate with the data collection platform (10);
identify, via the communication interface (102) according to the communication protocol, an address assigned to each of the one or more monitoring devices (20), the one or more monitoring devices (20) comprising the new monitoring device (20);
send, to the user computing device (30), the identified address or addresses assigned to the one or more monitoring devices (20);
receive, from the user computing device (30), a user input indicating a new address to be assigned to the new monitoring device (20); and
assign, via the communication interface (102) according to the communication protocol, the new address to the new monitoring device (20); and
wherein the user computing device (30) is configured to:
display, in response to receiving the identified address or addresses from the processor (100) of the data collection platform (10), a first graphical user interface, GUI, including:
the identified address or addresses assigned to the one or more monitoring devices (20), and
information to prompt a user to change the address assigned to the new monitoring device (20);
receive, via the first GUI, the user input to assign the new address to the new monitoring device (20); and
send, to the processor (100) of the data collection platform (10), the user input indicating the new address.

14. A data collection platform (10) adapted to collect environment data obtained by one or more monitoring devices (20), the data collection platform (10) comprising:
a communication interface (102) for communicating with the one or more monitoring devices (20) according to a communication protocol; and
a processor (100) configured to:
receive, from a user computing device (30) in communication with the data collection platform (10), an instruction to configure a new monitoring devic (20)e to communicate with the data collection platform (10);
automatically identify, via the communication interface (102) according to the communication protocol, an address assigned to each of the one or more monitoring devices (20), the one or more monitoring devices (20) comprising the new monitoring device (20);
send, to the user computing device (30), the identified address or addresses assigned to the one or more monitoring devices (20), thereby causing the user computing device (30) to display a first graphical user interface, GUI, including:
the identified address or addresses assigned to the one or more monitoring devices (20), and
information to prompt a user to change the address assigned to the new monitoring device (20);
receive, from the user computing device (30), a user input received by the user computing device (30) via the first GUI, the user input indicating a new address to be assigned to the new monitoring device (20); and
assign, via the communication interface (102) according to the communication protocol, the new address to the new monitoring device (20).

15. A user computing device (30) configured to communicate with a data collection platform (10) adapted to collect environment data obtained by one or more monitoring devices (20), the user computing device (30) comprising:
one or more processor (300)s configured to:
send, to the data collection platform (10), an instruction to configure a new monitoring device (20) to communicate with the data collection platform (10), thereby causing the data collection platform (10) to automatically identify, via a communication interface (102) according to a communication protocol, an address of each of the one or more monitoring devices (20), the one or more monitoring devices (20) comprising the new monitoring device (20);
receive, from the data collection platform (10), the identified address or addresses assigned to the one or more monitoring devices (20);
display a first graphical user interface, GUI, including:
the identified address or addresses assigned to the one or more monitoring devices (20), and
information to prompt a user to change the address assigned to the new monitoring device (20);
receive, via the first GUI, a user input to assign a new address to the new monitoring device (20); and
send, to the processor (100) of the data collection platform (10), the user input indicating the new address, thereby causing the data collection platform (10) to assign the new address to the new monitoring device (20).

## Amended claims

### Amended claims in accordance with Rule 137(2) EPC.

1. A method (500) performed by a processor (100) of a data collection platform (10) for configuring the data collection platform (10), the data collection being adapted to collect environment data obtained by one or more monitoring devices (20), the data collection platform (10) comprising the processor (100) and a communication interface (102) for communicating with the one or more monitoring devices (20) according to a serial communication protocol for intelligent sensors, the method comprising:
receiving, from a user computing device (30) in communication with the data collection platform (10), an instruction to configure a new monitoring device (20) to communicate with the data collection platform (10);
automatically identifying, via the communication interface (102) according to the communication protocol, an address of each of the one or more monitoring devices (20), the one or more monitoring devices (20) comprising the new monitoring device;
sending, to the user computing device (30), the identified address or addresses assigned to the one or more monitoring devices (20), thereby causing the user computing device (30) to display a first graphical user interface, GUI, including:
the identified address or addresses assigned to the one or more monitoring devices (20), and
information to prompt a user to change the address assigned to the new monitoring device (20);
receiving, from the user computing device (30), a user input received by the user computing device (30) via the first GUI, the user input indicating a new address to be assigned to the new monitoring device (20); and
assigning, via the communication interface (102) according to the communication protocol, the new address to the new monitoring device (20),
wherein the automatic identifying of the address assigned to each of the one or more monitoring devices (20) comprises:
enabling (531) power to the communication interface (102);
for each one of a plurality of possible addresses:
sending (532), via the communication interface (102) according to the communication protocol, a request to return identification information of a monitoring device (20) connected to the communication interface (102) under said one of the plurality of possible addresses;
receiving (533), via the communication interface (102) according to the communication protocol, a reply to the request;
in case the reply includes the identification information of the monitoring device (20), determining (534) that said one of the plurality of possible addresses is the address assigned to the monitoring device (20); and
in case the reply does not include the identification information, determining (535) that said one of the plurality of addresses is assigned to no monitoring device (20).

2. The method (500) of claim 1, the method comprising:
displaying (550), by the user computing device (30), the first graphical user interface, GUI, including:
the identified address or addresses assigned to the one or more monitoring devices (20), and
information to prompt a user to change the address assigned to the new monitoring device (20);
receiving (560), by the user computing device (30) via the first GUI, a user input to assign a new address to the new monitoring device (20); and
sending (570), by the user computing device (30) to the processor (100), the user input indicating the new address.

3. The method (500) according to claim 2, further comprising:
displaying, by the user computing device (30), a second GUI allowing the user to set configuration for the new monitoring device (20), the configuration including settings for one or more operations to be performed by the processor (100) with respect to the new monitoring device (20);
receiving (590), by the user computing device (30) via the second GUI, the configuration set by the user for the new monitoring device (20); and
sending the received configuration from the user computing device (30) to the data collection platform (10).

4. The method (500) according to claim 3, further comprising:
prior to the displaying of the second GUI, obtaining, by the user computing device (30) from a database, a template of configuration for the new monitoring device (20),
wherein the template comprises default settings of the configuration for the new monitoring device (20), and
wherein the second GUI shows the default settings included in the template.

5. The method (500) according to claim 4, wherein the default settings comprised in the template include settings for power control operations of the new monitoring device (20), specifying timings for turning on and/or off the new monitoring device (20).

6. The method (500) according to any one of claims 3 to 5, wherein the received configuration is sent to the data collection platform (10) in a specified format, and
wherein the specified format may be JSON or XML.

7. The method (500) according to any one of the preceding claims, further comprising: after the address assigned to each of the one or more monitoring devices (20 has been identified, disabling (538) power to the communication interface (102).

8. The method (500) according to any one of the preceding claims, wherein the assigning of the new address to the new monitoring device (20) comprises:
enabling (581) power to the communication interface (102); and
sending (582), via the communication interface (102) according to the communication protocol, a command to change the address identified for the new monitoring device (20) to the new address.

9. The method (500) according to claim 8, further comprising:
after the command to change the address has been effected, disabling (583) power to the communication interface (102).

10. The method (500) according to any one of the preceding claims, further comprising powering down the communication interface (102) when no further operations are required to be performed by the measuring devices (20).

11. The method (500) according to any one of the preceding claims, wherein the intelligent sensors monitor environment data, and
wherein the communication protocol may be:
Serial Digital Interface at 1200 baud, SDI-12,
Modbus, or
Universal Measurement Bus, UMB.

12. A computer program product comprising computer-readable instructions that, when loaded and run on a processor (100) comprised in a data collection platform (10), cause the processor (100) to perform the method (500) according to any one of the preceding claims.

13. A data collection platform (10) adapted to collect environment data obtained by one or more monitoring devices (20), the data collection platform (10) comprising:
a communication interface (102) for communicating with the one or more monitoring devices (20) according to a serial communication protocol for intelligent sensors; and
a processor (100) configured to perform the method of any one of claims 1, 7 and 8.

14. Data collection system comprising:
one or more monitoring devices (20) configured to obtain environment data;
the data collection platform (10) as defined in claim 13; and
a user computing device (30) configured to communicate with the data collection platform (10),
wherein the data collection platform (10) comprises:
a communication interface (102) for communicating with the one or more monitoring devices (20) according to a serial communication protocol for intelligent sensors; and
a processor configured to perform the user computing device (30) method steps of any of claims 2 to 6.
